# EUROPEAN PATENT SPECIFICATION

(11) **EP 3 875 067 B1**
(45) Date of publication and mention of the grant of the patent: **03.06.2026**
(21) Application number: 18939025.5
(22) Date of filing: 29.10.2018
(51) Int. Cl.: A61F 13/494, A61F 13/15, A61F 13/49

(54) **ABSORBENT ARTICLE**
ABSORBIERENDER ARTIKEL
ARTICLE ABSORBANT

(43) Date of publication of application: 08.09.2021
(73) Proprietor: Kao Corporation, Chuo-ku Tokyo 103-8210 (JP)
(72) Inventor: KAWAGUCHI, Hiroko, Haga-gun, Tochigi 321-3497 (JP); MATSUDA, Kohei, Haga-gun, Tochigi 321-3497 (JP); TADAI, Tomohiro, Haga-gun, Tochigi 321-3497 (JP)
(74) Representative: Vossius & Partner Patentanwälte Rechtsanwälte mbB
(86) International application number: PCT/JP2018/040137
(87) International publication number: WO 2020/089973

(56) References cited:
- WO-A1-2016/013448
- WO-A1-2018/168561
- CN-U- 206 138 289
- JP-A- 2003 199 788
- JP-A- 2003 500 165
- JP-A- 2009 207 660
- JP-A- 2011 156 110
- JP-A- 2011 206 218
- JP-A- 2013 116 196
- JP-A- 2016 036 519
- JP-A- 2016 112 208
- JP-A- 2018 153 571

## Description

### FIELD OF THE INVENTION

The present invention relates to an absorbent article.

### BACKGROUND OF THE INVENTION

Conventionally, proposals have been made on technologies for improving a wear feeling or leak-proofness for an absorbent article such as a diaper and the like.

For example, JP-A-2016-36519 describes a pants type diaper having a lengthwise long absorbent main unit and an outer cover, in which a longitudinal end portion of the absorbent main unit on a skin-contacting surface side is covered with an edge nonwoven fabric. From a viewpoint of enhancing comfort of putting on the pants type diaper, the end portion of the absorbent main unit is prevented from adhering to the outer cover and the edge nonwoven fabric. In a position which avoids the end portion of the absorbent main unit, the edge nonwoven fabric is adhered thereto wholly over a crosswise direction of the diaper on the skin-contacting surface side of the absorbent main unit, and the skin-contacting surface side of the outer cover.

JP-A-2016-112208 describes a pants type diaper in which a pair of leak-proof cuffs which extend in a longitudinal direction are joined to an absorbent main unit on skin-contacting surface sides thereof in a front portion and a rear portion from a viewpoint of liquid leakage prevention. In this pants type diaper, a covering sheet covers longitudinal end portion regions of the absorbent main unit and the leak-proof cuff. An end portion region of the covering sheet, which is positioned on a crotch portion side, is adjusted to be non-joined with the absorbent main unit and the leak-proof cuff wholly over a crosswise direction.

Further background is described in CN 206 138 289 U.

### SUMMARY OF INVENTION

The present invention provides an absorbent article as defined in independent claim 1.

The edge cover sheet may have, in a region adjacent to the weak adhesion portion on a longitudinal outward side of the absorbent main unit, a longitudinal strong adhesion portion having adhesive strength stronger than adhesive strength in the weak adhesion portion.

Preferbly, the weak adhesion portion is disposed in overlapping with an arrangement position of an absorbent body included in the absorbent main unit.

Preferably, the strong adhesion portions disposed adjacently on both sides of the weak adhesion portion in the crosswise direction are disposed in overlapping with an arrangement position of components of a three-dimensional gather portion included in the absorbent main unit.

According to preferred embodiment, the edge cover sheet has, in a region adjacent to the weak adhesion portion on a longitudinal inward side of the absorbent main unit, an easily peelable portion having adhesive strength weaker than adhesive strength in the weak adhesion portion. More preferably, the easily peelable portion is a non-adhesion portion.

According to preferred embodiment, rib-shaped fixing portions each of which joins the edge cover sheet with the absorbent main unit are disposed adjacently on both sides of the weak adhesion portion in the crosswise direction in a manner inclined relative to the longitudinal direction of the absorbent main unit, and an end portion of the fixing portion positioned on a longitudinal outward side of the absorbent main unit is arranged closer to a crosswise central side of the absorbent main unit than an end portion of the fixing portion positioned on a longitudinal inward side of the absorbent main unit.

According to preferred embodiment, the weak adhesion portion is disposed in overlapping with an arrangement position of an absorbent body included in the absorbent main unit, and rigidity in a site of the absorbent body arranged in a position which overlaps with the weak adhesion portion is lower than rigidity in a site of the absorbent body adjacent to the former site on a longitudinal inward side of the absorbent main unit. More preferably, a basis weight in the site of the absorbent body arranged in the position which overlaps with the weak adhesion portion is smaller than a basis weight in the site of the absorbent body adjacent to the former site on the longitudinal inward side of the absorbent main unit. Alternatively, a density in the site of the absorbent body arranged in the position which overlaps with the weak adhesion portion is smaller than a density in the site of the absorbent body adjacent to the former site on the longitudinal inward side of the absorbent main unit. According to another alternative, the absorbent article comprises a site in which an absorbent core having an absorbent material constituting the absorbent body is not disposed in the site of the absorbent body arranged in the position which overlaps with the weak adhesion portion.

According to preferred embodiment, an elastic member which allows the edge cover sheet to expand and contract in the crosswise direction is not disposed in a region corresponding to the weak adhesion portion of the absorbent article, and the elastic member which allows the edge cover sheet to expand and contract in the crosswise direction is disposed on a longitudinal outward side of the absorbent main unit from the weak adhesion portion. Preferably, an elastic member having the strongest contraction force among the elastic members disposed on the longitudinal outward side of the absorbent main unit from the weak adhesion portion is disposed in a position closest to the weak adhesion portion.

According to preferred embodiment, the edge cover sheet has, on a longitudinal inward side of the absorbent main unit, a folding back end portion, and the folding back end portion is fixed adjacently on both sides of the weak adhesion portion in the crosswise direction. More preferably, an elastic member which allows the edge cover sheet to expand and contract in the crosswise direction is disposed between the folding back end portion and the weak adhesion portion.

According to preferred embodiment, a length of the weak adhesion portion in the longitudinal direction is 2 mm or more and 7 mm or less, preferably 3 mm or more, and more preferably 4 mm or more, and preferably 6 mm or less, and more preferably 5 mm or less.

According to preferred embodiment, a length of the weak adhesion portion in the crosswise direction is 30 mm or more and 100 mm or less, preferably 40 mm or more, and more preferably 50 mm or more, and preferably 90 mm or less, and more preferably 80 mm or less.

According to preferred embodiment, a difference between adhesive strength in the strong adhesion portion and adhesive strength in the weak adhesion portion is 0.5 N/50mm or more and 1.9 N/50mm or less, preferably 0.7 N/50mm or more, and more preferably 0.9 N/50mm or more, and preferably 1.7 N/50mm or less, and more preferably 1.5 N/50mm or less.

According to preferred embodiment, adhesive strength in the strong adhesion portion is 1.3 N/50mm or more and 2.7 N/50mm or less, preferably 1.5 N/50mm or more, and more preferably 1.7 N/50mm or more, and preferably 2.5 N/50mm or less, and more preferably 2.3 N/50mm or less.

According to preferred embodiment, adhesive strength in the weak adhesion portion is 0.2 N/50mm or more and 0.8 N/50mm or less, preferably 0.3 N/50mm or more, and more preferably 0.4 N/50mm or more, and preferably 0.7 N/50mm or less, and more preferably 0.6 N/50mm or less.

According to preferred embodiment, adhesive strength reducer is incorporated into a surface of the absorbent article to which the weak adhesion portion is adhered. More preferably, the adhesive strength reducer is contained in hydrophilic finishing agent. Alternatively, the adhesive strength reducer is formed of a silicone compound.

According to preferred embodiment, bulk softness of the edge cover sheet is 4 cN or more and 7 cN or less, preferably 4.5 cN or more, and more preferably 5 cN or more, and preferably 6.5 cN or less, and more preferably 6 cN or less.

According to preferred embodiment, the edge cover sheet has, in a region adjacent to the weak adhesion portion on a longitudinal outward side of the absorbent main unit, a longitudinal strong adhesion portion having adhesive strength stronger than adhesive strength in the weak adhesion portion, and in a state in which the weak adhesion portion is peeled from the absorbent main unit, a pitch of a wrinkle of the weak adhesion portion is larger than a pitch of a wrinkle of the longitudinal strong adhesion portion.

According to preferred embodiment, the edge cover sheet has, in the region adjacent to the weak adhesion portion on the longitudinal outward side of the absorbent main unit, a longitudinal strong adhesion portion having adhesive strength stronger than adhesive strength in the weak adhesion portion, and in a state in which the weak adhesion portion is peeled from the absorbent main unit, a depth of a wrinkle of the weak adhesion portion is larger than a depth of a wrinkle of the longitudinal strong adhesion portion.

More preferably, a difference between adhesive strength in the longitudinal strong adhesion portion and adhesive strength in the weak adhesion portion is 0.5 N/50mm or more and 1.9 N/50mm or less, preferably 0.7 N/50mm or more, and more preferably 0.9 N/50mm or more, and preferably 1.7 N/50mm or less, and more preferably 1.5 N/50mm or less.

More preferably, adhesive strength in the longitudinal strong adhesion portion is 1.3 N/50mm or more and 2.7 N/50mm or less, preferably 1.5 N/50mm or more, and more preferably 1.7 N/50mm or more, and preferably 2.5 N/50mm or less, and more preferably 2.3 N/50mm or less.

According to preferred embodiment, the edge cover sheet is formed of a nonwoven fabric independent of the outer cover.

According to preferred embodiment, the weak adhesion portion is disposed in a position corresponding to the rear side of the wearer of the absorbent article.

Other and further objects, features and advantages of the invention will appear more fully from the following description, appropriately referring to the accompanying drawings.

### BRIEF DESCRIPTION OF DRAWINGS

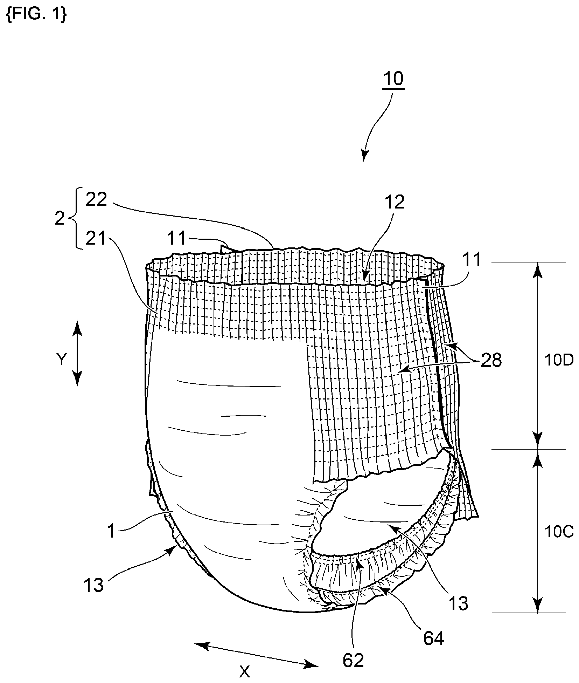

{FIG. 1}
   FIG. 1 is a perspective view of a pants type diaper showing a preferable embodiment of an absorbent article according to the invention.
{FIG. 2}
   FIG. 2 is a partial cutaway development plan view obtained by viewing, from a skin-contacting surface side, a state in which the pants type diaper shown in FIG. 1 is broken in side seal portions, and developed and elongated.
{FIG. 3}
   FIG. 3 is a partially enlarged plane view showing a weak adhesion portion and strong adhesion portions of an edge cover sheet in a rear portion of the pants type diaper shown in FIG. 2.
{FIG. 4}
   FIG. 4 is an explanatory view schematically showing, in a partially enlarged manner, a state in which the weak adhesion portion of the edge cover sheet is peeled and lifted from a crosswise central region of the absorbent main unit, and a leak-proof wall is formed in a rear portion of the pants type diaper shown in FIG. 2.
{FIG. 5}
   FIG. 5 is a partially enlarged plane view showing an aspect in which a longitudinal strong adhesion portion is disposed in a region adjacent to the weak adhesion portion on a longitudinal outward side of the absorbent main unit in a rear portion of the pants type diaper shown in FIG. 2.
{FIG. 6}
   FIG. 6 is a partially enlarged plane view showing modified examples (A) and (B) of arrangement regions of the strong adhesion portions in a rear portion of the pants type diaper shown in FIG. 2.
{FIG. 7}
   FIG. 7 is a partially enlarged plane view showing an aspect in an easily peelable portion is disposed in a region adjacent to the weak adhesion portion on a longitudinal inward side of the absorbent main unit in a rear portion of the pants type diaper shown in FIG. 2.
{FIG. 8}
   FIG. 8 is a partially enlarged plane view showing an aspect in which a rib-shaped fixing portion which joins the edge cover sheet with the absorbent main unit is disposed in a rear portion of the pants type diaper shown in FIG. 2.
{FIG. 9}
   FIG. 9(A) is a partially enlarged cross-sectional view showing an aspect in which rigidity in a site of an absorbent body disposed in a position which overlaps with a weak adhesion portion of an edge cover sheet is adjusted to be lower than rigidity in a site of an absorbent body adjacent thereto in a crosswise central region of a rear portion of a pants type diaper, and FIG. 9(B) is a partially enlarged cross-sectional view schematically showing a state in which the weak adhesion portion in FIG. 9(A) is peeled and lifted, and a leak-proof wall is formed.
{FIG. 10}
   FIG. 10 is a partially enlarged cross-sectional view schematically showing an arrangement relationship between a weak adhesion portion and an elastic member disposed in an outer cover in a crosswise central region of a rear portion of a pants type diaper.
{FIG. 11}
   FIG. 11(A) is a partially enlarged cross-sectional view showing an aspect in which an edge cover sheet has a folding back end portion in a rear portion of a pants type diaper, and FIG. 11(B) is a partially enlarged cross-sectional view showing an aspect in which an elastic member is disposed between the folding back end portion and the weak adhesion portion in FIG. 11(A).
{FIG. 12}
   FIG. 12 is a development plane view showing a modified example of an outer cover in a pants type diaper.

### DESCRIPTION OF EMBODIMENTS

The present invention relates to an absorbent article that suppresses an excreted fluid such as urine, loose stool and the like from leaking from an end portion on a front side or a rear side.

In the absorbent article such as a diaper and the like, measures for preventing an excreted fluid such as urine, loose stool and the like from side leak from a crotch have been conventionally taken by a three-dimensional gather portion or the like in which an elastic member is disposed. However, a wearer does not always turn the crotch downward, and may occasionally take a lying attitude. If the wearer is an infant or a disadvantaged person, a time during which the wearer is lying tends to be long, in particular. In this case, depending on an amount of the excreted fluid, the excreted fluid received in a crotch portion of the absorbent article may be led to flow to a front portion or a rear portion. Leading to leakage occurs more easily as a wearing time of the absorbent article becomes longer. At that time, leakage can be prevented by tightening the portion with a waist-surrounding gather portion in the diaper, for example. Even then, an anxiety of leakage still remains depending on a wearer's attitude and an amount of excretion, and a further improvement in leak-proofness against the excreted fluid is desired.

In contrast thereto, the absorbent article according to this invention can suppress the excreted fluid such as urine, loose stool and the like from leaking from an end portion on a front side or a rear side.

Hereinafter, a pants type (pull-on type) diaper 10 as a preferred embodiment of the absorbent article according to the present invention will be explained, referring to the drawings.

In the present invention, unless particularly stated otherwise, a side that is brought into contact with the human body is referred to as skin-facing surface side, skin-contacting surface side, or top surface side, and a side that is opposite to the above-mentioned side is referred to as non-skin-facing surface side, non-skin-contacting surface side, or back surface side. These terms are used as terms representing the relative positional relation in the member configuration of the absorbent article, even in connection with members that do not have a surface that is brought into contact with the human body. The direction positioned on the frontal side of the human body at the time of wearing is referred to as front, and the direction positioned on the rear side is referred to as rear. The direction normal to the top surface or the back surface of the absorbent article is referred to as thickness direction.

As shown in FIG. 1, a pants type diaper 10 (hereinafter, also referred to as a diaper 10) according to the embodiment has a lengthwise long absorbent main unit 1 and an outer cover 2. The outer cover 2 has a front outer portion 21 and a rear outer portion 22 corresponding to a front side and a rear side of a wearer, respectively. The front outer cover 21 and the rear outer portion 22 are laterally long, and respective edges on both sides are overlapped, and joined to each other in side seal portions 11, and an annular waist-surrounding portion 10D in the diaper 10 is formed. The absorbent main unit 1 extends from the outer cover 2 to form a crotch portion 10C in the diaper 10. Thus, the diaper 10 has a waist opening portion 12 in which an upper side of the waist-surrounding portion 10D is opened, and a pair of leg opening portions 13 and 13 in which the diaper 10 is opened on both left and right sides of the crotch portion 10C. According to the embodiment, the rear outer portion 22 extends toward the crotch portion 10C side from the side seal portion 11, which serves as a buttocks cover portion which covers wearer's buttocks. The side seal portion 11 can be formed by an ordinarily used means such as heat seal, ultrasonic seal and the like.

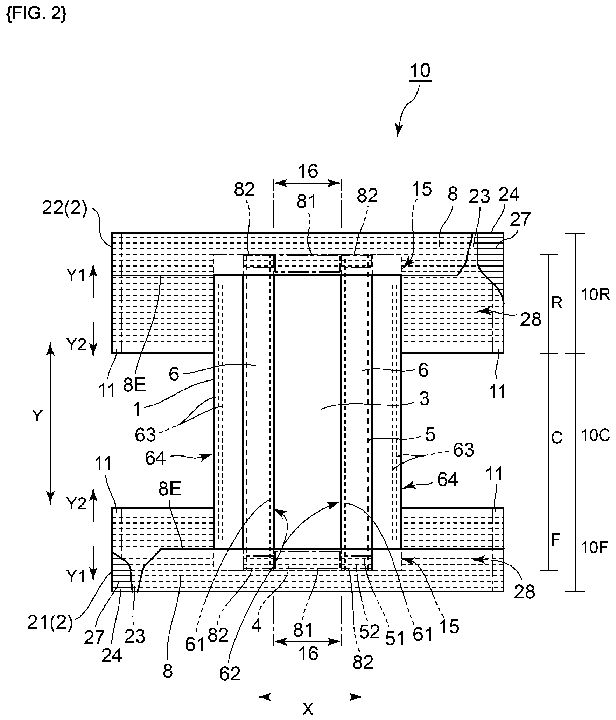

FIG. 2 shows a developed state in which the diaper 10 is broken in the side seal portions 11 and 11. The term "the developed state" herein means a state in which each portion is elongated to spread the absorbent article in a plane shape.

The diaper 10 is divided, in the developed state, into a front portion 10F, a crotch portion 10C, and a rear portion 10R. In the front portion 10F and the rear portion 10R, the laterally long front outer portion 21 and the laterally long rear outer portion 22 are disposed in a corresponding manner, respectively. The lengthwise long absorbent main unit 1 is disposed by directing a longitudinal direction thereof toward the front portion 10F, the crotch portion 10C, and the rear portion 10R of the diaper 10. In this arrangement, the absorbent main unit 1 has a longitudinal direction Y corresponding to a direction in which the front side, a crotch side, and the rear side of the wearer are linked, and a crosswise direction X perpendicular to the longitudinal direction Y. In corresponding to this wearer's site, the absorbent main unit 1 is longitudinally divided into a front portion F, a crotch portion C, and a rear portion R. The front portion F and the rear portion R of the absorbent main unit 1 are fixed to the skin-contacting surface sides of the front outer portion 21 and the rear outer portion 22, respectively. The crotch portion C of the absorbent main unit 1 configures the crotch portion 10C of the diaper 10. A developed shape of this diaper 10 has an I shape by the laterally long front outer portion 21 and the laterally long rear outer portion 22, and the lengthwise long absorbent main unit 1.

The crosswise direction X in the absorbent main unit 1 corresponds to a crosswise direction in the diaper 10 and the outer cover 2. The longitudinal direction Y of the absorbent main unit 1 corresponds to a direction of body length in the diaper 10 and the outer cover 2. At the time of merely referring to as "the longitudinal direction", the longitudinal direction means the longitudinal direction in the absorbent main unit 1, and the direction of body height of the diaper 10 and the outer cover 2.

In the diaper 10, a relative position of any other site to a certain site in the longitudinal direction Y may be occasionally referred to as on "a longitudinal outward Y1 side of the absorbent main unit 1" or on "a longitudinal inward Y2 side of the absorbent main unit 1". When any other site is on a side of a direction Y1 from the crotch portion C toward the front portion F or the rear portion R on seeing from a certain site, the other site is referred to as on "the longitudinal outward Y1 side of the absorbent main unit 1" from the certain site. When any other site is on a side of a direction Y2 from the front portion F or the rear portion R toward the crotch portion C on seeing from a certain site, the other site is referred to as on "the longitudinal inward Y2 side of the absorbent main unit 1" from a certain site. "The longitudinal outward Y1 side of the absorbent main unit 1" or "the longitudinal inward Y2 side of the absorbent main unit 1" is used as an indication showing a positional relationship between the sites in various components of the diaper 10 without limiting to the absorbent main unit 1. For example, as an indication showing a relative positional relationship in the longitudinal direction Y between sites other than the absorbent main unit 1 or between a site other than the absorbent main unit 1 and a site in the absorbent main unit 1, "the longitudinal outward Y1 side of the absorbent main unit 1" and "the longitudinal inward Y2 side of the absorbent main unit 1" as described above may be occasionally used.

Next, a configuration of the outer cover 2 and the absorbent main unit 1 is described.

The front outer portion 21 and the rear outer portion 22 which configure the outer cover 2 are formed of an inner layer sheet 23 on the skin-contacting surface side, and an outer layer sheet 24 on the non-skin-contacting surface side, respectively. Both the inner layer sheet 23 and the outer layer sheet 24 are preferably a nonwoven fabric which is substantially hydrophobic and has permeability of water vapor. Between this inner layer sheet 23 and this outer layer sheet 24, a waist-surrounding elastic member 27 is fixed in a state of being elongated in a waist-surrounding direction. The elastic member moderately expands and contracts in the waist-surrounding direction during wearing the diaper 10 to form a waist-surrounding gather portion 28, thereby fitting the diaper 10 to a wearer's waist-surrounding.

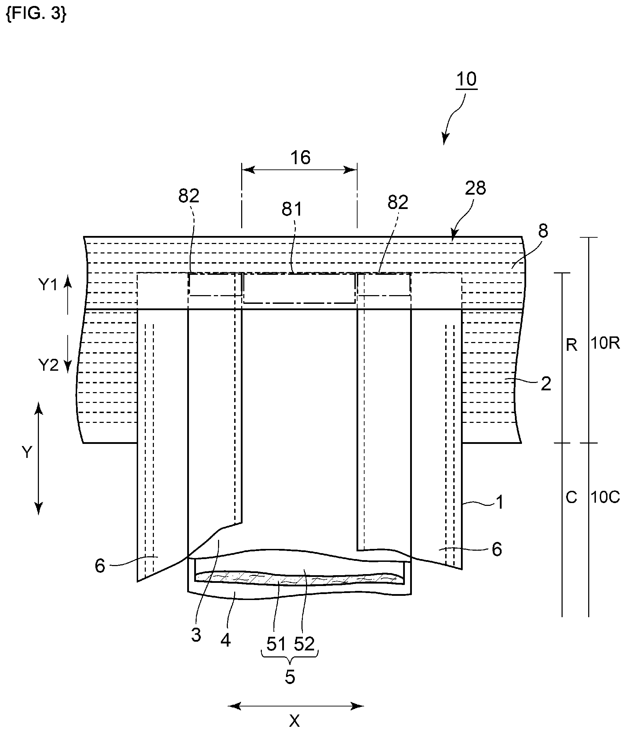

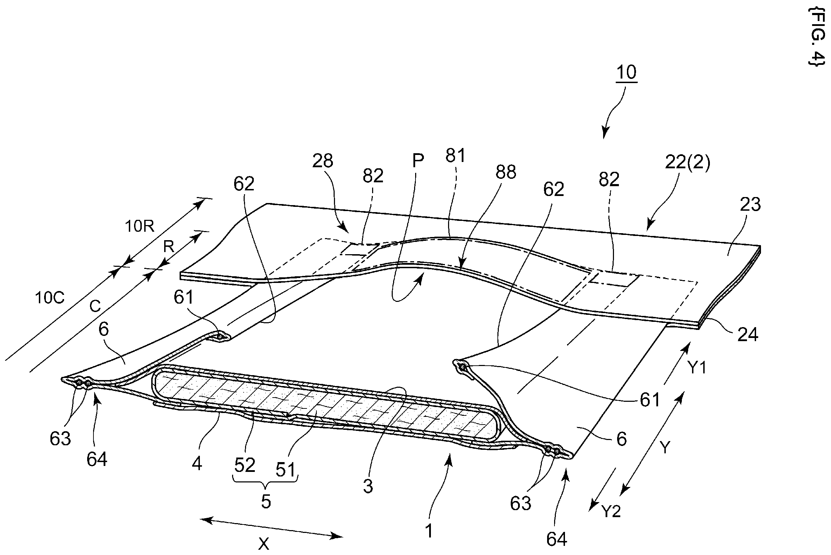

The absorbent main unit 1 has a fluid-permeable topsheet 3 on the skin-contacting surface side, a leak-proof backsheet 4 on the non-skin-contacting surface side, and a fluid-holding absorbent body 5 to be arranged between the topsheet 3 and the backsheet 4 (see FIG. 3 and FIG. 4). The absorbent body 5 has an absorbent core 51 formed of an aggregate of pulp fibers, or an aggregate of the pulp fibers and a superabsorbent polymer material, and a core wrap sheet 52 which covers an outer periphery of the absorbent core 51. From a viewpoint of improving texture, a nonwoven fabric sheet may be disposed on the non-skin-contacting surface side of the backsheet 4 (not shown).

The absorbent main unit 1 further has a pair of side sheets 6 on both sides of the absorbent main unit 1 in the crosswise direction X thereof. The side sheet 6 has, on the upper side on the skin-contacting surface side in each of regions on both sides of the topsheet 1 along the longitudinal direction Y thereof, a folding back end portion in which an elastic member 61 is fixed in an elongated state in the longitudinal direction Y. This part has stretchability to serve as a three-dimensional gather portion 62 which rises toward skin. The three-dimensional gather portion 62 prevents the excreted fluid from side leak thereof in the crotch portion 10C. Members (the side sheet 6 and the elastic member 61) which constitute the three-dimensional gather portion 62 are adhered, in a lodged manner, on a surface on the skin-contacting surface side of the absorbent main unit 1 in the front portion F and the rear portion R of the absorbent main unit 1. Thus, the three-dimensional gather portion 62 is prevented from rising in the front portion F and the rear portion R of the absorbent main unit 1. In addition, the side sheet 6 has, in a crosswise outward of the three-dimensional gather portion 62, a folding back end portion which is fixed in a state in which an elastic member 63 is elongated in the longitudinal direction Y. This part serves as a leg gather portion 64 in the crotch portion 10C.

On the skin-contacting surface side of the absorbent main unit 1, an edge cover sheet 8 which covers a surrounding of a longitudinal end portion 15 of the absorbent main unit 1 in the crosswise direction X is disposed. The edge cover sheet 8 covers each of the surroundings of the end portions 15 and 15 of the front portion F and the rear portion R of the absorbent main unit 1. As the edge cover sheet 8, a material which is used in an article of this kind can be used. From a viewpoint of improving the texture, the edge cover sheet 8 is preferably a nonwoven fabric. The edge cover sheet 8 may be a separate sheet independent of the outer cover 2, or a part in which the member which constitutes the outer cover 2 (the front outer portion 21 and the rear outer portion 22) extends in the direction of body length (corresponding to the longitudinal direction Y) of the diaper 10, and is folded back. According to the embodiment, the edge cover sheet 8 is applied as the separate sheet independent of the outer cover 2.

The edge cover sheet 8 has a length which extends in the longitudinal direction and in the crosswise direction from the front portion F and the rear portion R of the absorbent main unit 1 to the outer cover 2. Extended parts of the edge cover sheets 8 and 8 is adhered to the outer cover 2 (the front outer portion 21 and the rear outer portion 22). This adhesion can be performed by various methods which are used in the article of this kind. For example, the adhesion can be performed by coating a hot-melt adhesive.

As shown in FIG. 3, the edge cover sheet 8 has, in a position which overlaps with the absorbent main unit 1, a weak adhesion portion 81 and a strong adhesion portion 82 which have a difference in adhesive strength with the absorbent main unit 1. The weak adhesion portion 81 is disposed in a crosswise central region 16 of the absorbent main unit 1. The strong adhesion portions 82 are disposed adjacently on both sides of this weak adhesion portion 81 in the crosswise direction. That is, the weak adhesion portion 81 of the edge cover sheet and the strong adhesion portions 82 of the edge cover sheet on both sides thereof align in the crosswise direction X of the absorbent main unit 1, and are arrayed adjacently to each other. Although FIG. 3 shows the rear portion R of the absorbent main unit 1, the weak adhesion portion 81 and the strong adhesion portions 82 of the edge cover sheet 8 are aligned and disposed in the crosswise direction X similarly in the front portion F, too (also the same as in aspects shown below).

The terms "the weak adhesion portion 81" and "the strong adhesion portion 82" herein are specified on the basis of external force in association with ordinary movement such as pulling up the pants type diaper 10 on the upper side of the direction of body length upon putting on the pants type diaper 10. A weakly adhered part (the part with weak adhesive strength) at a degree at which the part is peeled off by the external force serves as "the weak adhesion portion 81" of the edge cover sheet 8. A strongly adhered part (the part with strong adhesive strength) which is not peeled off by the external force serves as "the strong adhesion portion 82". "The weak adhesion portion 81" and "the strong adhesion portion 82" can be formed by application of an adhesive which is ordinarily used. For example, the portions can be formed by differentiating the adhesive strength by changing an application basis weight or an application pattern of the hot-melt adhesive. The above-described pulling-up force is a force of 0.8 N/50mm or more and 1.3 N/50mm or less. According to the present invention, "the weak adhesion portion 81" and "the strong adhesion portion 82" are defined by measuring the adhesive strength by the following method.

### (Method for measuring adhesive strength)

The front portion 10F and the rear portion 10R of the diaper 10 is cut out in a size of 80 mm × 50 mm, centering on a site of a measurement object in a state in which the edge cover sheet 8 is adhered thereto. At this time, the site is cut out so as to include the weak adhesion portion 81 disposed in the crosswise central region 16 of the absorbent main unit 1 and the strong adhesion portions 82 adjacently on both sides. When a longitudinal strong adhesion portion 83 and an easily peelable portion 84 as described later, which are adjacent to the weak adhesion portion 81 in the longitudinal direction, are present, the site is cut out with including the parts.

On the cut-out site, a direction coinciding with the direction of body length of the diaper 10 is applied as a tensile direction, and the edge cover sheet and the absorbent main unit are chucked, and then a tensile test is performed under the following conditions:
temperature and relative humidity: 23°C, 65% R.H.,
tester: manufactured by Shimadzu Corporation, Autograph AGS-J (trade name),
interchuck distance: 20 mm,
tensile speed: 300 mm/min, and
tensile length: 50 mm.

In the above-described test, three pieces of the same samples are provided therefor and measured, and a mean of measured values is applied as the adhesive strength in the site of the measurement object.

From a viewpoint of more effectively developing the following action, a difference between the adhesive strength in the strong adhesion portion 82 and the adhesive strength in the weak adhesion portion 81 is 0.5 N/50mm or more, preferably 0.7 N/50mm or more, and further preferably 0.9 N/50mm or more. From a viewpoint of preventing the texture of the adhesion portion from deteriorating, the difference between the adhesive strength is preferably 1.9 N/50mm or less, more preferably 1.7 N/50mm or less, and further preferably 1.5 N/50mm or less. Specifically, the difference between the adhesive strength is preferably 0.5 N/50mm or more and 1.9 N/50mm or less, more preferably 0.7 N/50mm or more and 1.7 N/50mm or less, and further preferably 0.9 N/50mm or more and 1.5 N/50mm or less.

From the same viewpoint described above, the adhesive strength in the strong adhesion portion 82 is 1.3 N/50mm or more, preferably 1.5 N/50mm or more, and further preferably 1.7 N/50mm or more. From the same viewpoint described above, the adhesive strength in the strong adhesion portion 82 is preferably 2.7 N/50mm or less, more preferably 2.5 N/50mm or less, and further preferably 2.3 N/50mm or less. Specifically, the adhesive strength in the strong adhesion portion 82 is preferably 1.3 N/50mm or more and 2.7 N/50mm or less, more preferably 1.5 N/50mm or more and 2.5 N/50mm or less, and further preferably 1.7 N/50mm or more and 2.3 N/50mm or less.

From the same viewpoint described above, the adhesive strength in the weak adhesion portion 81 is 0.8 N/50mm or less, preferably 0.7 N/50mm or less, and further preferably 0.6 N/50mm or less. From the same viewpoint described above, the adhesive strength in the weak adhesion portion 81 is preferably 0.2 N/50mm or more, more preferably 0.3 N/50mm or more, and further preferably 0.4 N/50mm or more. Specifically, the adhesive strength in the weak adhesion portion 81 is preferably 0.2 N/50mm or more and 0.8 N/50mm or less, more preferably 0.3 N/50mm or more and 0.7 N/50mm or less, and further preferably 0.4 N/50mm or more and 0.6 N/50mm or less.

In the diaper 10, the weak adhesion portion 81 and the strong adhesion portions 82 and 82 of the edge cover sheet 8 align in the crosswise direction X, and are adhered to the surrounding of the end portion of the front portion F and the rear portion R of the absorbent main unit 1. Upon pulling up the diaper 10 by holding the waist opening portion 12 or the like in order to put on the diaper 10, the weak adhesion portion 81 of the edge cover sheet 8 is peeled off from the absorbent main unit 1 to perform action of lifting up toward wearer's skin. On the other hand, the strong adhesion portions 82 adjacently on both sides of the weak adhesion portion 81 in the crosswise direction maintain adhesion with the absorbent main unit 1. According to the embodiment, the edge cover sheet 8 is the separate sheet independent of the outer cover 2. Thus, the above-described action of lifting up of the weak adhesion portion 81 can be produced more clearly, and such a case is preferable.

Thus, as shown in FIG. 4, the weak adhesion portion 81 interposed between two strong adhesion portions 82 and 82 forms, in the crosswise central region 16 of the absorbent main unit 1, a pocket-shaped leak-proof wall 88 having a space portion P. The leak-proof wall 88 rises toward a wearer's skin surface to protect against the excreted fluid which is led to flow from an inward of the absorbent main unit 1 to the longitudinal outward Y1 side inside the space portion P. That is, the leak-proof wall 88 suppresses the excreted fluid from flowing to the waist-surrounding gather portion 28 of the outer cover 2 in which the absorbent main unit 1 is not disposed. The excreted fluid of which flow is blocked can be immediately absorbed and held in the absorbent body 5 in which absorption power is relatively kept in the crosswise central region 16 of the surrounding of the end portion 15 of the absorbent main unit 1. As a result, irrespective of the wearer's attitude or the amount of excretion, the excreted fluid can be prevented from leaking from a gap between gathers of the waist-surrounding gather portion 28 of the outer cover 2. Adhesion of the excreted fluid to the skin can be prevented also in a region in which the waist-surrounding gather portion 28 abuts on the skin. FIG. 4 shows the aspect by omitting the waist-surrounding elastic member 27 and the gathers of the waist-surrounding gather portion 28.

FIG. 4 shows a state in which the weak adhesion portion 81 forms the leak-proof wall 88 in the rear portion R. However, the weak adhesion portion 81 and the strong adhesion portions 82 and 82 are not limited only to the aspect in the rear portion R of the absorbent main unit 1, and may be arranged also in the front portion F. Thus, the leak-proof walls 88 and 88 against the excreted fluid which is led to flow can be formed in both the front portion F and the rear portion R of the absorbent main unit 1. However, because the rear side is turned downward in many cases when the wearer lies, the weak adhesion portion 81 and the strong adhesion portions 82 and 82 which align in the crosswise direction X are preferably arranged at least in a position corresponding to the rear side of the wearer (the rear portion of the absorbent main unit 1).

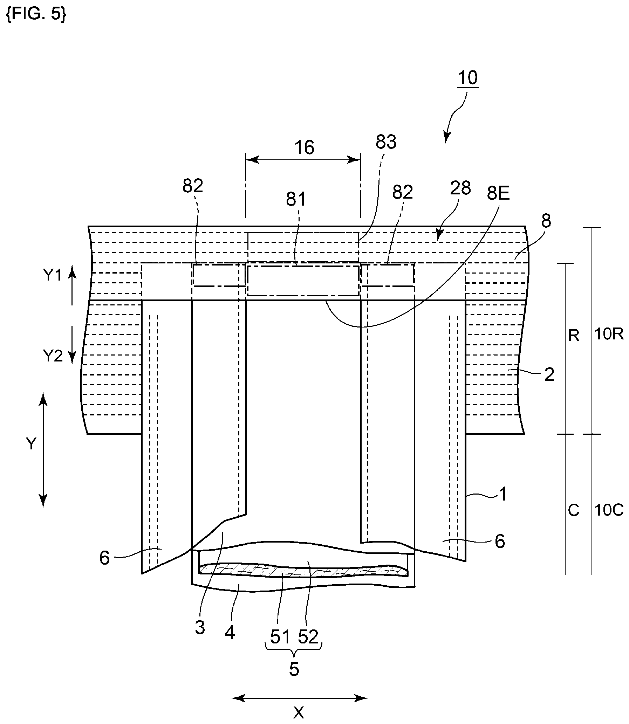

As shown in the aspect of FIG. 5, the edge cover sheet 8 preferably has, in a region adjacent to the weak adhesion portion 81 on the longitudinal outward Y1 side of the absorbent main unit 1, a longitudinal strong adhesion portion 83 in which the adhesive strength is stronger than the adhesive strength in the weak adhesion portion 81. According to this aspect, a size of the leak-proof wall 88 can be preferably controlled. That is, upon pulling up the diaper 10, the pocket-shaped space portion P can be prevented from spreading to a region of the outer cover 2 in which the absorbent main unit 1 is not disposed (for example, in the vicinity of the waist opening 12) by contagious peeling off of the weak adhesion portion 81 of the edge cover sheet 8. Thus, the size of the leak-proof wall 88 can be preferably controlled. Lifting up of the edge cover sheet 8 can be suppressed from being excessive, and satisfaction of both the leak-proofness against the excreted fluid and a wearer's wearing feeling can be further enhanced. From this viewpoint, the longitudinal strong adhesion portion 83 is preferably disposed in a position corresponding to the outer cover 2 on the longitudinal outward side of the absorbent main unit 1. The longitudinal strong adhesion portion 83 is more preferably disposed over part of the longitudinal end portion 15 of the absorbent main unit 1 from the position corresponding to the outer cover 2 on the longitudinal outward side of the absorbent main unit 1.

From a viewpoint of more effectively developing the above-described action, a difference between adhesive strength in the longitudinal strong adhesion portion 83 and the adhesive strength in the weak adhesion portion 81 is preferably 0.5 N/50mm or more, more preferably 0.7 N/50mm or more, and further preferably 0.9 N/50mm or more. From a viewpoint of preventing the texture of the adhesion portion from deteriorating, the difference between adhesive strength is preferably 1.9 N/50mm or less, more preferably 1.7 N/50mm or less, and further preferably 1.5 N/50mm or less. Specifically, the difference between adhesive strength is preferably 0.5 N/50mm or more and 1.9 N/50mm or less, more preferably 0.7 N/50mm or more and 1.7 N/50mm or less, and further preferably 0.9 N/50mm or more and 1.5 N/50mm or less.

From the same viewpoint described above, the adhesive strength in the longitudinal strong adhesion portion 83 is preferably 1.3 N/50mm or more, more preferably 1.5 N/50mm or more, and further preferably 1.7 N/50mm or more. From the same viewpoint described above, the adhesive strength in the longitudinal strong adhesion portion 83 is preferably 2.7 N/50mm or less, more preferably 2.5 N/50mm or less, and further preferably 2.3 N/50mm or less. Specifically, the adhesive strength in the longitudinal strong adhesion portion 83 is preferably 1.3 N/50mm or more and 2.7 N/50mm or less, more preferably 1.5 N/50mm or more and 2.5 N/50mm or less, and further preferably 1.7 N/50mm or more and 2.3 N/50mm or less.

From a viewpoint of more surely developing a fluid leakage prevention function by the leak-proof wall 88, the weak adhesion portion 81 is preferably disposed in overlapping with an arrangement position of the absorbent body 5 included in the absorbent main unit 1. Thus, the excreted fluid can be quickly absorbed into the absorbent body 5 from a position of protection against outflow of the excreted fluid in the leak-proof wall 88. The weak adhesion portion 81 is preferably arranged closer to the longitudinal inward Y2 side of the absorbent main unit 1 than the longitudinal outward Y1 side thereof in the edge cover sheet 8. In this case, the weak adhesion portion 81 may arrive at an end edge 8E on the longitudinal inward Y2 side of the edge cover sheet 8, or may be disposed on the longitudinal outward Y1 side in separating from the end edge 8E. The arrangement position of the absorbent body 5 is included in the crosswise central region 16 of the above-mentioned absorbent main unit 1.

From a viewpoint of more surely and more effectively forming the leak-proof wall 88, a length of the weak adhesion portion 81 in the longitudinal direction Y is preferably 2 mm or more, more preferably 3 mm or more, and further preferably 4 mm or more. From a viewpoint of suppressing lifting up of the edge cover sheet 8 from being excessive, the length of the weak adhesion portion 81 in the longitudinal direction Y is preferably 7 mm or less, more preferably 6 mm or less, and further preferably 5 mm or less. Specifically, the length of the weak adhesion portion 81 in the longitudinal direction Y is preferably 2 mm or more and 7 mm or less, more preferably 3 mm or more and 6 mm or less, and further preferably 4 mm or more and 5 mm or less.

From the same viewpoint, a length of the weak adhesion portion 81 in the crosswise direction X is preferably 30 mm or more, more preferably 40 mm or more, and further preferably 50 mm or more. From a viewpoint of suppressing lifting up of the edge cover sheet 8 from being excessive, the length of the weak adhesion portion 81 in the crosswise direction X is preferably 100 mm or less, more preferably 90 mm or less, and further preferably 80 mm or less. Specifically, the length of the weak adhesion portion 81 in the crosswise direction X is preferably 30 mm or more and 100 mm or less, more preferably 40 mm or more and 90 mm or less, and further preferably 50 mm or more and 80 mm or less.

From a viewpoint of further enhancing the fluid leakage prevention function by the leak-proof wall 88, an area ratio of a region in which the weak adhesion portion 81 overlaps with the arrangement position of the absorbent body 5 in a whole region of the weak adhesion portion 81 is preferably 80% or more, more preferably 90% or more, and further preferably 100%.

The length of the weak adhesion portion 81 in the longitudinal direction Y and the length thereof in the crosswise direction X, and the area ratio as described above are expressed in terms of values obtained by breaking the diaper 10 in the side seal portions 11 and 11, and measuring each portion in a plane view in an elongated and developed state.

On the other hand, the strong adhesion portions 82 disposed adjacently on both sides of the weak adhesion portion 81 in the crosswise direction are preferably disposed in overlapping with an arrangement position of the components of the three-dimensional gather portion 62 of the absorbent main unit 1. Thus, the above-mentioned space portion P can be prevented from being formed up to the components (the side sheet 6 and the elastic member 61) of the three-dimensional gather portion 62 which is adhered thereto in the lodged manner. In addition, the leak-proof wall 88 which is formed of the weak adhesion portion 81 of the edge cover sheet 8, and a part in which the three-dimensional gather portion 62 rises are disposed adjacently in the front portion F and the rear portion R of the absorbent main unit 1, and leak-proof functions of both can be interlocked, and developed. For example, even when, while protecting against leading flow of the excreted fluid in the three-dimensional gather portion 62, the excreted fluid further diffuses in the longitudinal direction Y, the excreted fluid can be easily captured in the space portion P in the leak-proof wall 88 adjacent thereto. Thus, the excreted fluid which diffuses in the longitudinal direction Y along both sides of the absorbent main unit 1 can be guided into the crosswise central region 16, and can be quickly absorbed and held by the site of the absorbent body 5 in which the absorption power in the position is relatively kept.

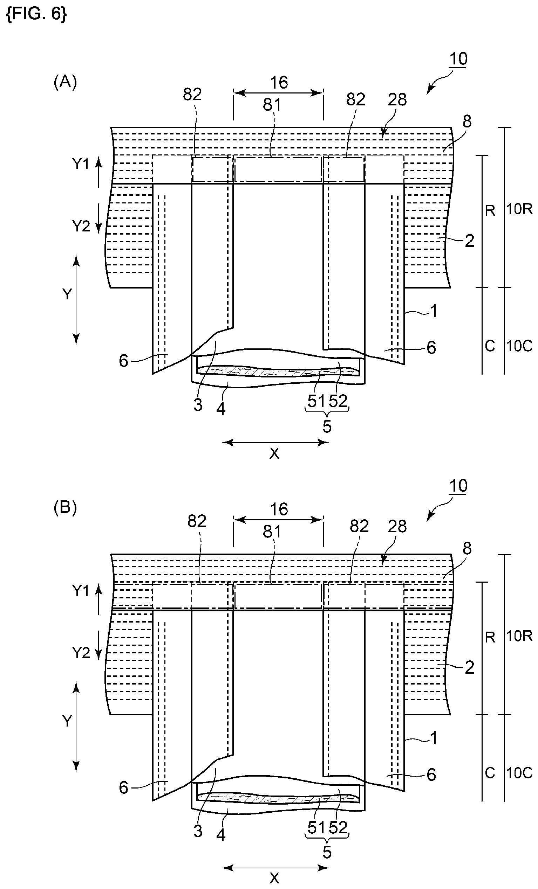

A length of the strong adhesion portion 82 in the longitudinal direction Y and a length thereof in the crosswise direction X are not limited to dimensions according to the aspects shown in FIGs. 3 to 5, and can be appropriately set. For example, as shown in FIG. 6(A), the length of the strong adhesion portion 82 in the longitudinal direction Y may be adjusted to be the same as the length of the weak adhesion portion 81 in the longitudinal direction Y. As shown in FIG. 6(B), the length of the strong adhesion portion 82 in the crosswise direction X may be a length extending from a position corresponding to the absorbent main unit 1 to a position corresponding to the outer cover 2 in a crosswise outward (the same as in the aspects shown below). In this case, the strong adhesion portion 82 is to be adhered to both the absorbent main unit 1 and the outer cover 2 with strong adhesive strength. Thus, both sides of the space portion P in the leak-proof wall 88 to be formed by the weak adhesion portion 81 can be surely closed, and such a case is preferable.

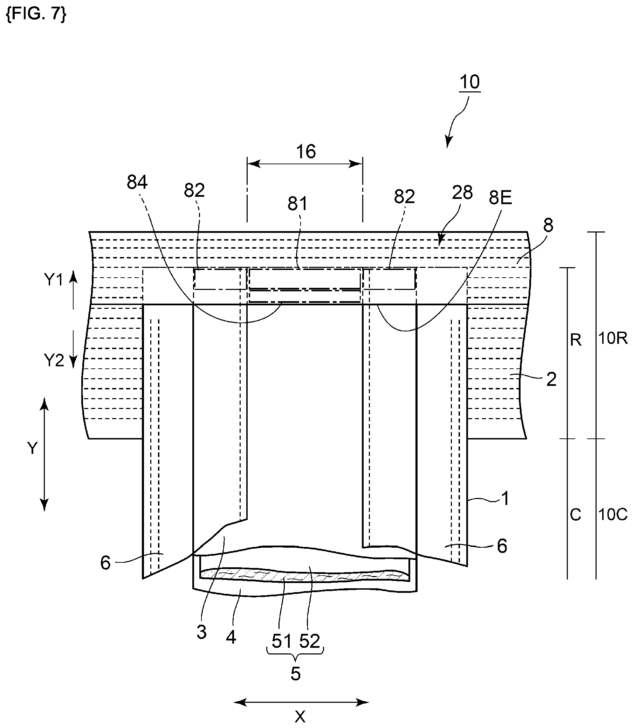

As shown in the aspect of FIG. 7, the edge cover sheet 8 preferably has, in a region adjacent to the weak adhesion portion 81 on the longitudinal inward Y2 side of the absorbent main unit 1, an easily peelable portion 84 in which adhesive strength is weaker than the adhesive strength in the weak adhesion portion 81. In the case of this aspect, the easily peelable portion 84 is arranged in abutting on the end edge 8E on the longitudinal inward Y2 side of the edge cover sheet 8. The weak adhesion portion 81 is to be disposed in separating, by a portion of the easily peelable portion 84, on the longitudinal outward Y1 side from the end edge 8E. That is, the easily peelable portion 84 and the weak adhesion portion 81 are disposed in this order from the end edge 8E of the edge cover sheet 8 on the longitudinal inward Y2 side thereof toward a longitudinal outward Y1. Thus, the edge cover sheet 8 can be peeled off in two steps, and the size of the leak-proof wall 88 can be preferably controlled. Lifting up of the edge cover sheet 8 can be suppressed from being excessive, and satisfaction of both the leak-proofness against the excreted fluid and the wearer's wearing feeling can be further enhanced. Also in this case, the above-mentioned longitudinal strong adhesion portion 83 is preferably disposed.

The easily peelable portion 84 may be a part to which the adhesive is coated, or a non-adhesion portion to which the adhesive is not coated. From a viewpoint of more surely forming the leak-proof wall 88, the easily peelable portion 84 is preferably the non-adhesion portion. The easily peelable portion 84 in the non-adhesion portion acts as a trigger of peeling of the edge cover sheet 8 upon pulling up the diaper 10. When the easily peelable portion 84 is the part to which the adhesive is coated, the adhesive strength thereof is preferably 0.5 N/50mm or less, more preferably 0.4 N/50mm or less, and further preferably 0.3 N/50mm or less. From a viewpoint of acting as the trigger of peeling of the edge cover sheet 8 upon pulling up the diaper 10, the adhesive strength of the easily peelable portion 84 in this case is preferably 0.1 N/50mm or more, more preferably 0.15 N/50mm or more, and further preferably 0.2 N/50mm or more. Specifically, the adhesive strength of the easily peelable portion 84 is preferably 0.1 N/50mm or more and 0.5 N/50mm or less, more preferably 0.15 N/50mm or more and 0.4 N/50mm or less, and further preferably 0.2 N/50mm or more and 0.3 N/50mm or less.

From a viewpoint of more surely and more effectively forming the leak-proof wall 88, a length of the easily peelable portion 84 in the longitudinal direction Y is preferably 3 mm or more, more preferably 5 mm or more, and further preferably 7 mm or more. From a viewpoint of preventing appearance from deteriorating, the length of the easily peelable portion 84 in the longitudinal direction Y is preferably 15 mm or less, more preferably 13 mm or less, and further preferably 10 mm or less. Specifically, the length of the easily peelable portion 84 in the longitudinal direction Y is preferably 3 mm or more and 15 mm or less, more preferably 5 mm or more and 13 mm or less, and further preferably 7 mm or more and 10 mm or less.

When the easily peelable portion 84 has the above-described length in the longitudinal direction Y, the length of the weak adhesion portion 81 in the longitudinal direction Y can be appropriately set in a range of falling within the region corresponding to the absorbent main unit 1 (the region without exceeding the longitudinal end edge of the absorbent main unit 1) within the above-mentioned preferable range.

A length of the easily peelable portion 84 in the crosswise direction X is preferably the same as or less than the length of the weak adhesion portion 81 in the crosswise direction X. When the length of the easily peelable portion 84 in the crosswise direction X is less than the length of the weak adhesion portion 81 in the crosswise direction X, the easily peelable portion 84 is preferably disposed in overlapping with the arrangement position of the absorbent body 5. The easily peelable portion 84 is preferably disposed in extending from a crosswise center of the absorbent main unit 1 to right and left. Specifically, from a viewpoint of more surely and more effectively forming the leak-proof wall 88, the length of the easily peelable portion 84 in the crosswise direction X is preferably 30 mm or more, more preferably 40 mm or more, and further preferably 50 mm or more. From a viewpoint of acting as the trigger of peeling of the edge cover sheet 8 upon pulling up the diaper 10, the length of the easily peelable portion 84 in the crosswise direction X is preferably 70 mm or less, more preferably 65 mm or less, and further preferably 60 mm or less. More specifically, the length of the easily peelable portion 84 in the crosswise direction X is preferably 30 mm or more and 70 mm or less, more preferably 40 mm or more and 65 mm or less, and further preferably 50 mm or more and 60 mm or less.

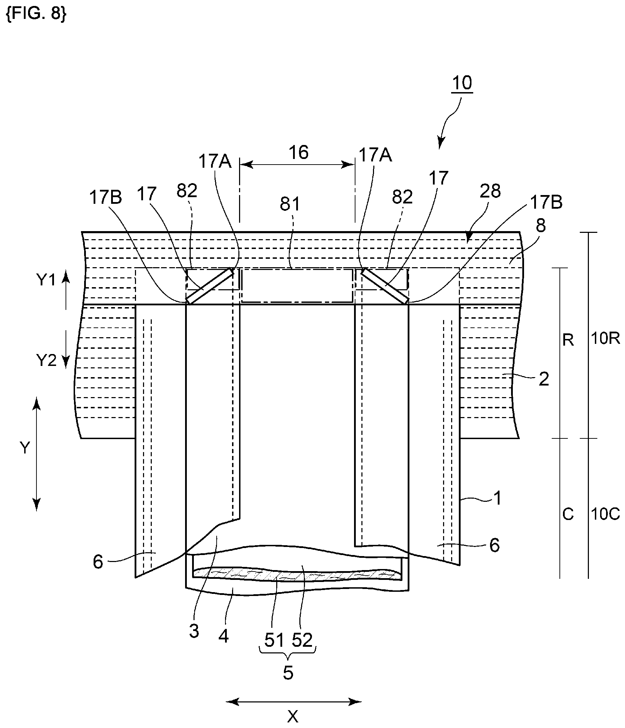

In addition, as shown in the aspect of FIG. 8, a pair of fixing portions 17 and 17 between the edge cover sheet 8 and the absorbent main unit 1 are preferably disposed adjacently on both sides of the weak adhesion portion 81 in the crosswise direction. The fixing portion 17 is formed into a rib shape in the plane view of the edge cover sheet 8, and a rib-shaped part is disposed in a manner inclined relative to the longitudinal direction of the absorbent main unit. The fixing portion 17 which extends in the rib shape has an end portion 17A and an end portion 17B. The end portion 17A is positioned on the longitudinal outward Y1 side of the absorbent main unit 1, and the end portion 17B is positioned on the longitudinal inward Y2 side of the absorbent main unit 1. The end portion 17A of the fixing portion 17, which is positioned on the longitudinal outward Y1 side of the absorbent main unit 1, is arranged closer to a crosswise central side of the absorbent main unit than the end portion 17B of the fixing portion 17, which is positioned on the longitudinal inward Y2 side of the absorbent main unit 1.

More specifically, the following arrangement is formed upon the plane view of the edge cover sheet 8. That is, the pair of fixing portions 17 and 17 is arranged on both sides of the weak adhesion portion 81 in the crosswise direction X by interposing the region of the weak adhesion portion 81. The end portions 17A and 17A with regard to each fixing portion 17 are arranged in facing each other, and the end portions 17B and 17B are arranged in facing each other. A separation distance between the end portions 17A and 17A is adjusted to be shorter than a separation distance between the end portions 17B and 17B.

According to this aspect, the weak adhesion portion 81 of the edge cover sheet 8 is easily peeled off in a U shape (that is, an area in which the weak adhesion portion 81 is peeled off toward the longitudinal outward Y1 side of the absorbent main unit 1 is easily narrowed) to enhance the leak-proofness against the excreted fluid. Lifting up of the edge cover sheet 8 can be suppressed from being excessive, and satisfaction of both the leak-proofness against the excreted fluid and the wearer's wearing feeling can be further enhanced.

From a viewpoint of the above-described action, the fixing portion 17 is preferably disposed in overlapping with an arrangement position of the strong adhesion portion 82 of the edge cover sheet 8. The fixing portion 17 is preferably disposed in overlapping with the arrangement position of the components of the three-dimensional gather portion 62 of the absorbent main unit 1.

The fixing portion 17 may be formed by applying the adhesive between the edge cover sheet 8 and the absorbent main unit 1, or may be formed by performing compression bonding by emboss treatment of the edge cover sheet 8 and the absorbent main unit 1. When the fixing portion 17 is formed by using the adhesive, the fixing portion 17 may be formed of a coating portion of the adhesive which forms the strong adhesion portion 82 of the edge cover sheet 8, or may be a coating portion separate from the coating portion of the adhesive which forms the strong adhesion portion 82. When the fixing portion 17 is formed of the coating portion of the adhesive which forms the strong adhesion portion 82, the strong adhesion portion 82 is to be formed along a shape of the fixing portion 17. When the fixing portion 17 is formed by emboss treatment, the fixing portion 17 is formed as the coating portion separate from the coating portion of the adhesive which forms the strong adhesion portion 82 of the edge cover sheet 8.

If the fixing portion 17 is a compression-bonded portion by emboss treatment, it is unnecessary to take account of oozing of the adhesive, and the fixing portion 17 can be formed up to the end edge 8E on the longitudinal inward Y2 side of the edge cover sheet 8, and therefore such a case is preferred.

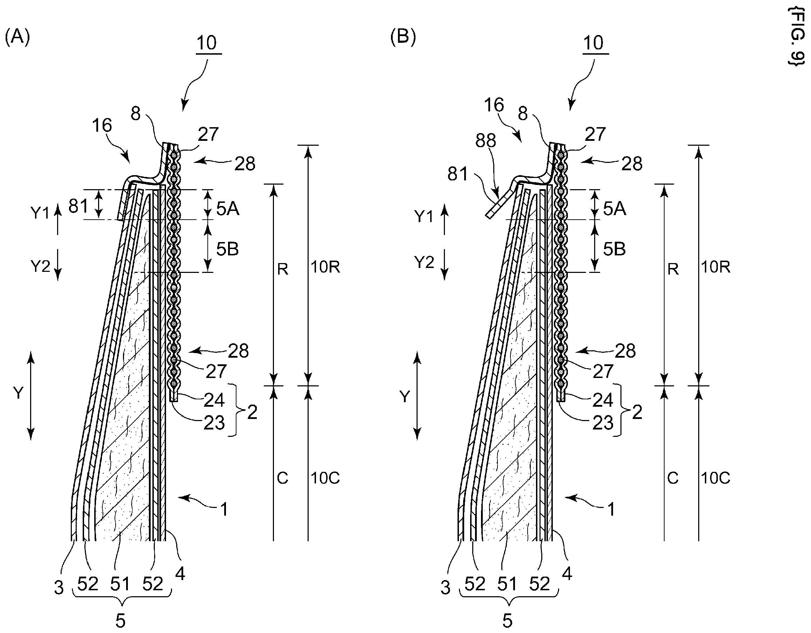

The diaper 10 preferably has an aspect in which the weak adhesion portion 81 overlaps with the arrangement position of the absorbent body 5 of the absorbent main unit 1, and also overlaps with an arrangement position of a site in which rigidity of the absorbent body 5 is relatively low. Specifically, the rigidity in a site 5A of the absorbent body 5 arranged in a position which overlaps with the weak adhesion portion 81 is preferably lower than rigidity in a site 5B of the absorbent body 5 adjacent to the site 5A on the longitudinal inward Y2 side of the absorbent main unit 1. A specific example includes, as shown in the aspect of FIG. 9(A), a case where a thickness of the absorbent body 5 is formed into a shape of reducing the thickness from the longitudinal inward Y2 side of the absorbent main unit 1 to the longitudinal outward Y1 side thereof. As shown in the aspect of FIG. 9(A), from a viewpoint of holding absorbency of the absorbent body, a degree of reduction is preferably in a gentle inclination. The inclination according to which the thickness is reduced is preferably formed in a range of one-third of a longitudinal length of the absorbent body 5 in the front portion and the rear portion of the absorbent body 5.

In this case, the rigidity of the absorbent body 5 corresponding to the weak adhesion portion 81 of the edge cover sheet 8 is relatively low. Thus, as shown in FIG. 9(B), the weak adhesion portion 81 more easily contracts by contraction force of the waist-surrounding elastic member 27 of the outer cover 2, and the above-mentioned leak-proof wall 88 easily rises on a wearer's skin side. The outer cover 2 expands and contracts according to movement of the wearer such as the infant and the like, and the leak-proof wall 88 by the weak adhesion portion 81 easily follows the wearer's skin in interlocking therewith to easily closely contact with the wearer's skin. Thus, a gap is hard to be formed between a risen leak-proof wall 88 and the skin, and leakage becomes more difficult to occur.

The site 5A in low rigidity of the absorbent body 5 corresponding to the weak adhesion portion 81 can be formed by a basis weight, a density or the like of the absorbent body, in addition to the above-described thickness of the absorbent body.

That is, a specific example includes a case where a basis weight in the site 5A of the absorbent body 5 arranged in the position which overlaps with the weak adhesion portion 81 is adjusted to be smaller than a basis weight in the site 5B of the absorbent body 5 adjacent to the site 5A on the longitudinal inward Y2 side of the absorbent main unit 1. A specific example includes a case where a density in the site 5A of the absorbent body 5 arranged in the position which overlaps with the weak adhesion portion 81 is adjusted to be smaller than a density in the site 5B of the absorbent body 5 adjacent to the site 5A on the longitudinal inward Y2 side of the absorbent main unit 1.

A specific example includes an aspect in which a site to which an absorbent core 51 having an absorbent material (pulp fibers, a superabsorbent polymer material, or the like) constituting the absorbent body 5 is not disposed is incorporated into the site 5A of the absorbent body 5 arranged in the position which overlaps with the weak adhesion portion 81 (not shown).

An aspect is preferable in which the elastic member which allows the edge cover sheet 8 to expand and contract in the crosswise direction X is not disposed in a region of the diaper 10 corresponding to the weak adhesion portion 81. The elastic member which allows the edge cover sheet 8 to expand and contract in the crosswise direction X is preferably disposed on the longitudinal outward Y1 side of the absorbent main unit 1 than the weak adhesion portion 81. The region of the diaper 10 corresponding to the weak adhesion portion 81 herein means the region of the diaper 10 which overlaps with the weak adhesion portion 81 in the thickness direction. For example, this region includes the weak adhesion portion 81 per se of the edge cover sheet 8, and also a part interposed between the edge cover sheet 8 and the absorbent main unit 1, the absorbent main unit 1, a part interposed between the absorbent main unit 1 and the outer cover 2, and the outer cover 2. The elastic member which allows the edge cover sheet 8 to expand and contract in the crosswise direction X means the elastic member which is joined to a member in the elongated state. The elastic member is joined to the member in the elongated state to allow the member to expand and contract in association with expansion and contraction of the elastic member. However, if the elastic member which is joined to the member in the elongated state is in a state in which the member is cut off on the way to be discontinuous, the elastic member is not in the elongated state in the cut-off part, and cannot allow the member to expand and contract.

It is preferable that the elastic member in the elongated state is not disposed in the region of the diaper 10 corresponding to the weak adhesion portion 81, and is disposed on the longitudinal outward Y1 side of the absorbent main unit 1 from the weak adhesion portion 81. It should be noted that, even if the elastic member in a contracted state in the state in which the member is cut off on the way to be discontinuous is in a position of the weak adhesion portion 81, in this case, the elastic member which allows the edge cover sheet 8 to expand and contract in the crosswise direction X is not to be disposed in the region of the diaper 10 corresponding to the weak adhesion portion 81.

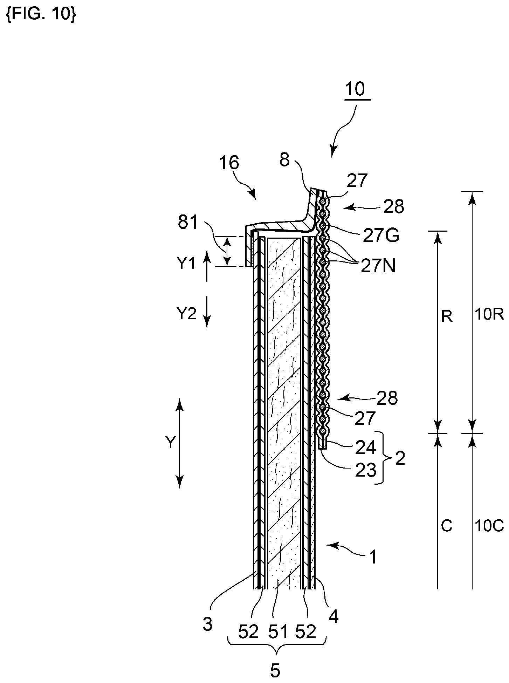

A specific example includes, as shown in the aspect of FIG. 10 in the embodiment, a waist-surrounding elastic member 27 which allows the outer cover 2 to expand and contract. The waist-surrounding elastic member 27 allows the outer cover 2 (the front outer portion 21 and the rear outer portion 22) to expand and contract, thereby allowing the edge cover sheet 8 joined to the outer cover 2 to expand and contract. With regard to this waist-surrounding elastic member 27, a waist-surrounding elastic member 27N adjusted in the state in which the member is cut off to be discontinuous is preferably disposed in the region of the outer cover 2 corresponding to the weak adhesion portion 81. In addition, the waist-surrounding elastic member 27 to be disposed on the longitudinal outward Y1 side of the absorbent main unit 1 from the weak adhesion portion 81 of the edge cover sheet 8 is preferably adjusted in a state in which the member 27 is not cut off to be continuous.

Thus, the edge cover sheet 8 on the longitudinal outward Y1 side of the absorbent main unit 1 from the weak adhesion portion 81 more easily contracts, and the leak-proof wall 88 more easily rises. The leak-proof wall 88 more easily rises by the elastic member, and therefore the leak-proof wall 88 easily follows the wearer's skin according to the movement of the wearer such as the infant, and easily closely contacts therewith. Thus, the gap is hard to be formed between the risen leak-proof wall 88 and the skin, and the leakage becomes more difficult to occur.

With regard to arrangement of the above-described elastic member, an aspect is preferable in which an elastic member having the strongest contraction force among the elastic members disposed on the longitudinal outward Y1 side of the absorbent main unit 1 from the weak adhesion portion 81 is disposed in a position closest to the weak adhesion portion 81. Specifically, as shown in the aspect of FIG. 10 in the embodiment, a waist-surrounding elastic member 27G in the position closest to the weak adhesion portion 81 preferably has the strongest contraction force. Thus, the above-described action by the elastic member can be more effectively developed.

In the aspect shown in FIG. 10, the diaper 10 more preferably has the above-mentioned aspect of the rigidity of the absorbent body 5. That is, in addition to the arrangement of the elastic member, the rigidity is more preferably smaller in the site 5A of the absorbent body 5 arranged in the position which overlaps with the weak adhesion portion 81 than in the site B of the absorbent body adjacent to the site 5A on the longitudinal inward Y2 side of the absorbent main unit 1.

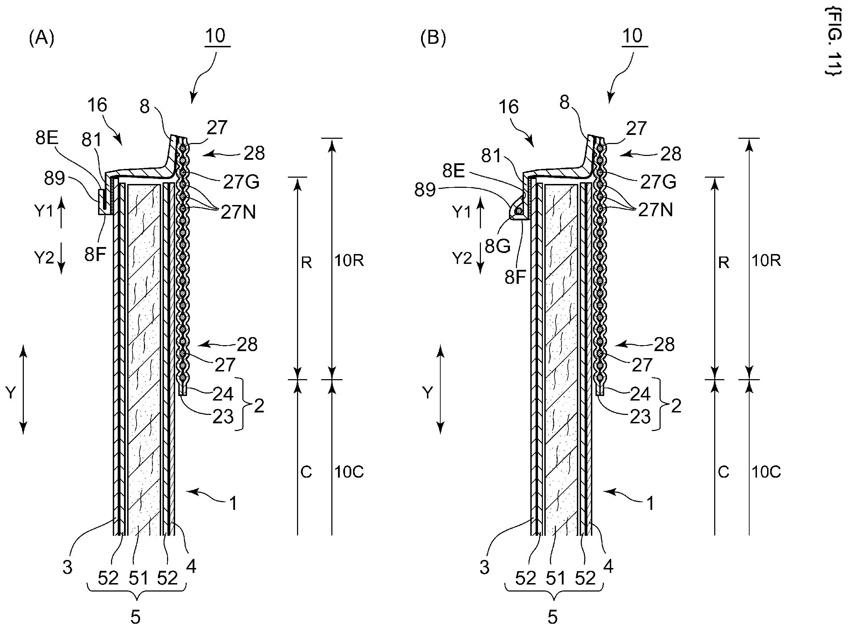

As shown in the aspect of FIG. 11(A) in the embodiment, the edge cover sheet 8 preferably has, on the longitudinal inward Y2 side of the absorbent main unit 1, a folding back end portion 89. The folding back end portion 89 is a part formed by folding back, toward the longitudinal outward Y1 side with a fold line 8F as an axis, a surrounding part including the end edge 8E (see FIG. 5) on the longitudinal inward Y2 side of the edge cover sheet 8. The folding back end portion 89 is a part without including the weak adhesion portion 81 of the edge cover sheet 8, and the fold line 8F is preferably disposed adjacently to an edge of the weak adhesion portion 81. This folding back end portion 89 may be wholly or partly in the crosswise direction X of the edge cover sheet 8. The folding back end portion 89 is preferably disposed at least in the region corresponding to the weak adhesion portion 81.

The folding back end portions 89 are preferably fixed adjacently on both sides of the weak adhesion portion 81 in the crosswise direction. This fixation can be formed by a method which is ordinarily used in the article of this kind, and specific examples include a method using an adhesive and a method by emboss treatment. This fixation may be combinedly formed by the adhesive with which the edge cover sheet 8 is adhered, or the above-mentioned fixing portion 17. In particular, the fixation is preferably formed in a position which overlaps with the components of the three-dimensional gather portion 62 of the absorbent main unit 1.

Thus, the weak adhesion portion 81 of the edge cover sheet 8 is more easily lifted, and the leak-proof wall 88 easily rises. As a result, the gap is hard to be formed between the risen leak-proof wall 88 and the skin, and the leakage becomes more difficult to occur.

As shown in the aspect of FIG. 11(B), from a viewpoint of more effectively developing the action by the above-described folding back end portion 89, an elastic member 8G which allows the edge cover sheet to expand and contract in the crosswise direction X is preferably disposed between the folding back end portion 89 and the weak adhesion portion 81, in particular, in the vicinity of the fold line 8F. In this case, the elastic member 8G is joined thereto, in the elongated state, in the crosswise direction X along the fold line 8F. The elastic member 8G is preferably disposed according to a length of the folding back end portion 89 in the crosswise direction X.

In the embodiment, the following aspect about the edge cover sheet 8 is preferable.

From a viewpoint of facilitating to peel off the weak adhesion portion 81, an adhesive strength reducer is preferably incorporated into a surface of the absorbent main unit 1 to which the weak adhesion portion 81 is adhered. As the adhesive strength reducer, the reducer which is ordinarily used in the article of this kind can be adopted without particular restriction. Specific examples include a solid mold release agent such as a fluorine-based mold release agent, a silicone-based mold release agent, and vaseline.

The adhesive strength reducer is preferably contained in hydrophilic finishing agent. Thus, when the edge cover sheet 8 is formed of the nonwoven fabric, the nonwoven fabric can be provided with hydrophilicity and mold releasability at one time, in which processability is improved, and simultaneously the fiber can be uniformly provided with the above properties. Therefore, an effect of satisfactorily forming the above-mentioned leak-proof wall 88 is enhanced. In particular, from a viewpoint of uniformly suspending the adhesive strength reducer in the hydrophilic finishing agent, the adhesive strength reducer is preferably formed of a silicone compound.

Bulk softness of the edge cover sheet 8 is preferably 7 cN or less, more preferably 6.5 cN or less, and further preferably 6 cN or less. Thus, rigidity of the edge cover sheet 8 is suppressed, and even if the edge cover sheet 8 forms the leak-proof wall 88, texture to the wearer is hard to deteriorate. From a viewpoint of enhancing peelability when the diaper 10 is pulled up, the bulk softness of the edge cover sheet 8 is preferably 4 cN or more, more preferably 4.5 cN or more, and further preferably 5 cN or more. Specifically, the bulk softness of the edge cover sheet 8 is preferably 4 cN or more and 7 cN or less, more preferably 4.5 cN or more and 6.5 cN or less, and further preferably 5 cN or more and 6 cN or less.

### (Method for measuring bulk softness)

The bulk softness of the edge cover sheet 8 can be measured by the following method. That is, under an environment of 22°C and 65% RH, the edge cover sheet 8 is cut out into a piece of 150 mm in the crosswise direction of the diaper, and 30 mm in the direction of body length of the diaper, and end portions thereof are stapled in two upper and lower positions by using a stapler into a ring shape having a diameter of 45 mm. At this time, a staple of the stapler is fixed to be long in the crosswise direction. The ring is set in a cylindrical form on a specimen stage to measure a maximum load upon compressing the ring at a rate of 10 mm/min in a compression rate by means of a flat plate substantially in parallel to the stage from above by using a tensile tester (for example, Tensilon tensile tester "RTA-100", manufactured by Orientec Co. Ltd.), and this measured value is taken as the bulk softness.

In a state in which the weak adhesion portion 81 of the edge cover sheet 8 is peeled from the absorbent main unit 1, a pitch of a wrinkle of the weak adhesion portion 81 is preferably larger than a pitch of a wrinkle of the above-mentioned longitudinal strong adhesion portion 83. Thus, the leak-proof wall 88 forms a large gather, and therefore closely contacts with the skin, and a leakage suppression effect is enhanced.

The term "the pitch of the wrinkle" means a distance, in the crosswise direction X, between top portions of parts protruded on the skin-contacting surface side with regard to the "wrinkle" which is formed after the leak-proof wall 88 is formed. The "wrinkle" means the wrinkle in the contracted state of the front portion 10F and the rear portion 10R of the diaper 10 to which the edge cover sheet 8 is adhered respectively. The term "contracted state" means a state in which the front portion 10F and the rear portion 10R are allowed to contract in the crosswise direction X from a state in which the front portion 10F and the rear portion 10R are elongated in the crosswise direction X at a maximum, in a state in which the diaper 10 is placed flat on a flat surface without applying a load to the diaper 10.

In the state in which the weak adhesion portion 81 of the edge cover sheet 8 is peeled from the absorbent main unit 1, a depth of the wrinkle of the weak adhesion portion 81 is preferably larger than a depth of the wrinkle of the above-mentioned longitudinal strong adhesion portion 83. Thus, the leak-proof wall 88 forms the large gather, and therefore closely contacts with the skin, and the leakage suppression effect is enhanced.

The term "the depth of the wrinkle" means a distance, in the thickness direction of the edge cover sheet 8, between a top portion and a bottom portion of a part protruded on the skin-contacting surface side with regard to the "wrinkle" which is formed after the leak-proof wall 88 is formed. The "wrinkle" means the wrinkle in the above-mentioned "contracted state".

### (Method for measuring pitch of wrinkle)

First, a material obtained by pulling up the diaper 10 on the upper side of the direction of body length (longitudinal direction Y) to peel the weak adhesion portion 81 of the edge cover sheet 8 is formed into the developed state and the elongated state, as shown in FIG. 2. Next, in each of the front portion 10F and the rear portion 10R of the diaper 10, an existence region of the peeled weak adhesion portion 81 is taken as a measurement range. In the front portion 10F, a red line extending linearly in the crosswise direction X is drawn in a center of the peeled weak adhesion portion 81 in the longitudinal direction Y. A red line extending linearly in the crosswise direction X is drawn in a center of the longitudinal strong adhesion portion 83 in the longitudinal direction Y. In the same manner, in the rear portion 10R, red lines extending linearly in the crosswise direction X are drawn in a center of the peeled weak adhesion portion 81 in the longitudinal direction Y, and in a center of the longitudinal strong adhesion portion 83 in the longitudinal direction Y, respectively.

After the diaper 10 in this state is left to stand for 24 hours under an environment of ordinary temperature (room temperature of about 18 to 22°C, and 55 to 65% relative humidity), the diaper 10 is released from the elongated state to allow the front portion 10F and the rear portion 10R to contract in the crosswise direction X. Thus, a plurality of wrinkles are formed in the weak adhesion portion 81 and the longitudinal strong adhesion portion 83 of the diaper 10 in the state in which the diaper 10 is placed flat in a plane shape. After the diaper 10 is left to stand for a while in an intact state, an interval between the top portions of the wrinkle which is formed on the red line in the measurement range and extends in the longitudinal direction Y is measured by a rule, a caliper or the like, for example. With regard to each of the weak adhesion portion 81 and the longitudinal strong adhesion portion 83, an interval which is maximized among the measured intervals serves as the pitch of the wrinkle of the weak adhesion portion 81 in the peeled state or the pitch of the wrinkle of the longitudinal strong adhesion portion 83.

### (Method for measuring depth of wrinkle)

In the (Method for measuring pitch of wrinkle), a distance, in the thickness direction, between the top portion and the bottom portion of the edge cover sheet on the skin-contacting surface side is measured on each of the "wrinkle" designated as the pitch of the wrinkle of the weak adhesion portion 81 in the peeled state and the "wrinkle" designated as the pitch of the wrinkle of the longitudinal strong adhesion portion 83 by using the rule, the caliper or the like, for example. Measured distances serve as the depth of the wrinkle of the weak adhesion portion 81 in the peeled state, and the depth of the wrinkle of the longitudinal strong adhesion portion 83, respectively. A depth which is maximized among the measured depths serves as the depth of the wrinkle of the weak adhesion portion 81 in the peeled state, or the depth of the wrinkle of the longitudinal strong adhesion portion 83.

In the pants type diaper 10 according to the embodiment, the aspect is not limited to a case where the diaper 10 is provided with each of the aspects listed above independently, and may be formed into a material provided with an aspect obtained by appropriately combining several aspects.

As described above, in the pants type diaper 10 according to the embodiment, the weak adhesion portion 81 of the edge cover sheet 8 is peeled when the diaper 10 is pulled up in the direction of body length to form the leak-proof wall 88 which rises toward the wearer's skin surface. Thus, the pants type diaper 10 according to the embodiment can effectively suppress the excreted fluid such as urine, loose stool and the like from leaking from the end portion on the front side or the rear side.

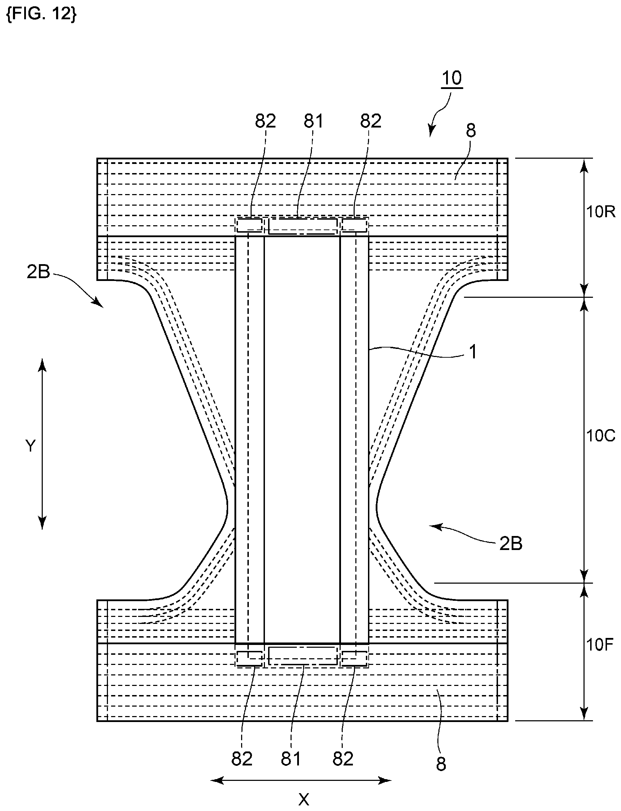

The pants type diaper 10 according to the embodiment may be appropriately formed into a product in another form in a shape of the outer cover 2 as long as the diaper 10 has the edge cover sheet 8 having the weak adhesion portion 81 which can form the leak-proof wall 88. For example, another form may be an outer cover 2B as shown in FIG. 12. The outer cover 2B has a size wholly over the front portion 10F, the crotch portion 10C, and the rear portion 10R of the diaper 10. The outer cover 2B has, in the developed state, a constricted plane shape in which a crosswise length in the crotch portion 10C is adjusted to be shorter than crosswise lengths in the front portion 10F and the rear portion 10R. The above-mentioned edge cover sheet 8 may be a material formed of the nonwoven fabric independent of the outer cover 2B, or may be a material in which the outer cover 2B extends in the longitudinal outward Y1, and is folded back.

The absorbent article according to the present invention may be the pants type diaper according to the above-described embodiment, and also a tape type diaper, or sanitary shorts, a shorts type napkin, incontinence underwear or the like in a concept of including the absorbent article having the front portion to be disposed on the front side of the wearer, and the rear portion to be disposed on the rear side of the wearer. Other members may also be appropriately incorporated into the above-described configuration according to an application or a function.

### DESCRIPTION OF SYMBOLS

1 Absorbent main unit
   15 Longitudinal end portion of absorbent main unit
   16 Crosswise central region of absorbent main unit
   17 Fixing portion
   17A, 17B End portion of fixing portion
2, 2B Outer cover
   21 Front outer portion
   22 Rear outer portion
   23 Inner layer sheet
   24 Outer layer sheet
   27 Waist-surrounding elastic member
   28 Waist-surrounding gather portion
3 Topsheet
4 Backsheet
5 Absorbent body
   51 Absorbent core
   52 Core wrap sheet
   5A Site in low rigidity of absorbent body
   5B Site in high rigidity of absorbent body
6 Side sheet
   61 Elastic member
   62 Three-dimensional gather portion
   63 Elastic member
   64 Leg gather portion
8 Edge cover sheet
   81 Weak adhesion portion
   82 Strong adhesion portion
   83 Longitudinal strong adhesion portion
   84 Easily peelable portion
   8E End edge on longitudinal inward side of edge cover sheet
   88 Leak-proof wall
   89 Folding back end portion
   P Pocket-shaped space portion
10 Pants type diaper
10F Front portion of pants type diaper
10C Crotch portion of pants type diaper
10R Rear portion of pants type diaper
F Front portion of absorbent main unit
C Crotch portion of absorbent main unit
R Rear portion of absorbent main unit
Y Longitudinal direction of absorbent main unit (or direction of body length of diaper and outer cover)
   Y1 Longitudinal outward of absorbent main unit
   Y2 Longitudinal inward of absorbent main unit
X Crosswise direction of absorbent main unit (or crosswise direction of diaper and outer cover)

## Claims

1. An absorbent article comprising a lengthwise long absorbent main unit (1), and an outer cover (2) disposed on a non-skin-contacting surface side of the absorbent main unit (1), wherein
the absorbent main unit (1) has a longitudinal direction (Y) corresponding to a direction in which a front side, a crotch side and a rear side of a wearer are linked, and a crosswise direction (X) perpendicular to the longitudinal direction (Y);
an edge cover sheet (8) which covers surrounding of a longitudinal end portion (15) of the absorbent main unit (1) in the crosswise direction (X) is disposed on a skin-contacting surface side of the absorbent main unit (1); and
the edge cover sheet (8) has a weak adhesion portion (81) and a strong adhesion portion (82) with the absorbent main unit (1),
the weak adhesion portion (81) is disposed in a crosswise central region (16) of the absorbent main unit (1) and has an adhesive strength of 0.8 N/50mm or less, and
the strong adhesion portions (82) are disposed adjacently on both sides of the weak adhesion portion (81) in the crosswise direction (X) and have an adhesive strength of 1.3 N/50mm or more;
wherein the adhesive strength is measured in accordance with the method of the description.

2. The absorbent article according to Claim 1, wherein the edge cover sheet (8) has, in a region adjacent to the weak adhesion portion (81) on a longitudinal outward side of the absorbent main unit (1), a longitudinal strong adhesion portion (83) having adhesive strength stronger than adhesive strength in the weak adhesion portion (81).

3. The absorbent article according to Claim 1 or 2, wherein the weak adhesion portion (81) is disposed in overlapping with an arrangement position of an absorbent body (5) included in the absorbent main unit (1).

4. The absorbent article according to any one of Claims 1 to 3, wherein the strong adhesion portions (82) disposed adjacently on both sides of the weak adhesion portion (81) in the crosswise direction (X) are disposed in overlapping with an arrangement position of components of a three-dimensional gather portion (62) included in the absorbent main unit (1).

5. The absorbent article according to any one of Claims 1 to 4, wherein the edge cover sheet (8) has, in a region adjacent to the weak adhesion portion (81) on a longitudinal inward side of the absorbent main unit (1), an easily peelable portion (84) having adhesive strength weaker than adhesive strength in the weak adhesion portion (81).

6. The absorbent article according to Claim 5, wherein the easily peelable portion (84) is a non-adhesion portion.

7. The absorbent article according to any one of Claims 1 to 6, wherein rib-shaped fixing portions (17) each of which joins the edge cover sheet (8) with the absorbent main unit (1) are disposed adjacently on both sides of the weak adhesion portion (81) in the crosswise direction (X) in a manner inclined relative to the longitudinal direction (Y) of the absorbent main unit (1), and
an end portion of the fixing portion (17) positioned on a longitudinal outward side of the absorbent main unit (1) is arranged closer to a crosswise central side of the absorbent main unit (1) than an end portion of the fixing portion (17) positioned on a longitudinal inward side of the absorbent main unit (1).

8. The absorbent article according to any one of Claims 1 to 7, wherein the weak adhesion portion (81) is disposed in overlapping with an arrangement position of an absorbent body (5) included in the absorbent main unit (1), and rigidity in a site of the absorbent body (5) arranged in a position which overlaps with the weak adhesion portion (81) is lower than rigidity in a site of the absorbent body (5) adjacent to the former site on a longitudinal inward side of the absorbent main unit (1).

9. The absorbent article according to Claim 8, wherein a basis weight in the site of the absorbent body (5) arranged in the position which overlaps with the weak adhesion portion (81) is smaller than a basis weight in the site of the absorbent body (5) adjacent to the former site on the longitudinal inward side of the absorbent main unit (1).

10. The absorbent article according to Claim 8, wherein a density in the site of the absorbent body (5) arranged in the position which overlaps with the weak adhesion portion (81) is smaller than a density in the site of the absorbent body (5) adjacent to the former site on the longitudinal inward side of the absorbent main unit (1).

11. The absorbent article according to Claim 8, comprising a site in which an absorbent core (51) having an absorbent material constituting the absorbent body (5) is not disposed in the site of the absorbent body (5) arranged in the position which overlaps with the weak adhesion portion (81).

12. The absorbent article according to any one of Claims 1 to 11, wherein an elastic member which allows the edge cover sheet (8) to expand and contract in the crosswise direction is not disposed in a region corresponding to the weak adhesion portion (81) of the absorbent article, and the elastic member which allows the edge cover sheet (8) to expand and contract in the crosswise direction (X) is disposed on a longitudinal outward side of the absorbent main unit (1) from the weak adhesion portion (81).

13. The absorbent article according to Claim 12, wherein an elastic member having the strongest contraction force among the elastic members disposed on the longitudinal outward side of the absorbent main unit (1) from the weak adhesion portion (81) is disposed in a position closest to the weak adhesion portion (81).

14. The absorbent article according to any one of Claims 1 to 13, wherein the edge cover sheet (8) has, on a longitudinal inward side of the absorbent main unit (1), a folding back end portion (89), and the folding back end portion (89) is fixed adjacently on both sides of the weak adhesion portion (81) in the crosswise direction (X).

15. The absorbent article according to Claim 14, wherein an elastic member which allows the edge cover sheet (8) to expand and contract in the crosswise direction (X) is disposed between the folding back end portion (89) and the weak adhesion portion (81).

## Patentansprüche

1. Absorbierender Artikel, aufweisend eine in Längsrichtung lange absorbierende Haupteinheit (1) und eine äußere Hülle (2), die auf einer nicht mit der Haut in Kontakt stehenden Oberflächenseite der absorbierenden Haupteinheit (1) angeordnet ist, wobei
die absorbierende Haupteinheit (1) eine Längsrichtung (Y), die einer Richtung entspricht, in der eine Vorderseite, eine Schrittseite und eine Rückseite eines Trägers miteinander verbunden sind, sowie eine Querrichtung (X) aufweist, die senkrecht zur Längsrichtung (Y) verläuft;
eine Randabdeckfolie (8), die den Umfang eines Längsendabschnitts (15) der absorbierenden Haupteinheit (1) in der Querrichtung (X) abdeckt, auf einer mit der Haut in Kontakt stehenden Oberflächenseite der absorbierenden Haupteinheit (1) angeordnet ist; und
die Randabdeckfolie (8) einen an der absorbierenden Haupteinheit (1) schwach haftenden Abschnitt (81) und einen an der absorbierenden Haupteinheit (1) stark haftenden Abschnitt (82) aufweist,
wobei der schwach haftende Abschnitt (81) in einem quer verlaufenden Mittelbereich (16) der absorbierenden Haupteinheit (1) angeordnet ist und eine Haftkraft von 0,8 N/50 mm oder weniger aufweist, und
die stark haftenden Abschnitte (82) in der Querrichtung (X) benachbart auf beiden Seiten des schwach haftenden Abschnitts (81) angeordnet sind und eine Haftkraft von 1,3 N/50 mm oder mehr aufweisen;
wobei die Haftkraft gemäß dem in der Beschreibung angegebenen Verfahren gemessen wird.

2. Absorbierender Artikel nach Anspruch 1, wobei die Randabdeckfolie (8) in einem Bereich benachbart zu dem schwach haftenden Abschnitt (81) auf einer Längsaußenseite der absorbierenden Haupteinheit (1) einen stark haftenden Längsabschnitt (83) aufweist, dessen Haftkraft stärker ist als die Haftkraft in dem schwach haftenden Abschnitt (81).

3. Absorbierender Artikel nach Anspruch 1 oder 2, wobei der schwach haftende Abschnitt (81) so angeordnet ist, dass er eine Anordnungsposition eines in der absorbierenden Haupteinheit (1) enthaltenen Absorptionskörpers (5) überlappt.

4. Absorbierender Artikel nach einem der Ansprüche 1 bis 3, wobei die stark haftenden Abschnitte (82), die in der Querrichtung (X) benachbart auf beiden Seiten des schwach haftenden Abschnitts (81) angeordnet sind, überlappend zu einer Anordnungsposition von Komponenten eines in der absorbierenden Haupteinheit (1) enthaltenen dreidimensionalen Raffabschnitts (62) angeordnet sind.

5. Absorbierender Artikel nach einem der Ansprüche 1 bis 4, wobei die Randabdeckfolie (8) in einem Bereich benachbart zu dem schwach haftenden Abschnitt (81) auf einer Längsinnenseite der absorbierenden Haupteinheit (1) einen leicht abziehbaren Abschnitt (84) aufweist, dessen Haftkraft schwächer ist als die Haftkraft in dem schwach haftenden Abschnitt (81).

6. Absorbierender Artikel nach Anspruch 5, wobei der leicht abziehbare Abschnitt (84) ein nicht haftender Abschnitt ist.

7. Absorbierender Artikel nach einem der Ansprüche 1 bis 6, wobei rippenförmige Befestigungsabschnitte (17), von denen jeder die Randabdeckfolie (8) mit der absorbierenden Haupteinheit (1) verbindet, benachbart auf beiden Seiten des schwach haftenden Abschnitts (81) in der Querrichtung (X) geneigt zur Längsrichtung (Y) der absorbierenden Haupteinheit (1) angeordnet sind, und
ein Endabschnitt des Befestigungsabschnitts (17), der an einer Längsaußenseite der absorbierenden Haupteinheit (1) angeordnet ist, näher an einer quer verlaufenden Mittelseite der absorbierenden Haupteinheit (1) angeordnet ist als ein Endabschnitt des Befestigungsabschnitts (17), der an einer Längsinnenseite der absorbierenden Haupteinheit (1) positioniert ist.

8. Absorbierender Artikel nach einem der Ansprüche 1 bis 7, wobei der schwach haftende Abschnitt (81) so angeordnet ist, dass er eine Anordnungsposition eines in der absorbierenden Haupteinheit (1) enthaltenen Absorptionskörpers (5) überlappt, und die Steifigkeit an einer Stelle des Absorptionskörpers (5), die an einer Position angeordnet ist, die den schwach haftenden Abschnitt (81) überlappt, geringer ist als die Steifigkeit an einer Stelle des Absorptionskörpers (5) benachbart zu der erstgenannten Stelle auf einer Längsinnenseite der absorbierenden Haupteinheit (1).

9. Absorbierender Artikel nach Anspruch 8, wobei ein Flächengewicht an der Stelle des Absorptionskörpers (5), die an der Position angeordnet ist, die den schwach haftenden Abschnitt (81) überlappt, geringer ist als ein Flächengewicht an der Stelle des Absorptionskörpers (5) benachbart zu der erstgenannten Stelle auf der Längsinnenseite der absorbierenden Haupteinheit (1).

10. Absorbierender Artikel nach Anspruch 8, wobei eine Dichte an der Stelle des Absorptionskörpers (5), die an der Position angeordnet ist, die den schwach haftenden Abschnitt (81) überlappt, geringer ist als eine Dichte an der Stelle des Absorptionskörpers (5) benachbart zu der erstgenannten Stelle auf der Längsinnenseite der absorbierenden Haupteinheit (1).

11. Absorbierender Artikel nach Anspruch 8, der eine Stelle aufweist, an der ein Absorptionskern (51) mit einem Absorptionsmaterial, das den Absorptionskörper (5) bildet, nicht an der Stelle des Absorptionskörpers (5) angeordnet ist, die an der Position angeordnet ist, die den schwach haftenden Abschnitt (81) überlappt.

12. Absorbierender Artikel nach einem der Ansprüche 1 bis 11, wobei ein elastisches Element, das es der Randabdeckfolie (8) ermöglicht, sich in der Querrichtung auszudehnen und zusammenzuziehen, nicht in einem Bereich angeordnet ist, der dem schwach haftenden Abschnitt (81) des absorbierenden Artikels entspricht, und das elastische Element, das es der Randabdeckfolie (8) ermöglicht, sich in der Querrichtung (X) auszudehnen und zusammenzuziehen, auf einer Längsaußenseite der absorbierenden Haupteinheit (1) von dem schwach haftenden Abschnitt (81) aus angeordnet ist.

13. Absorbierender Artikel nach Anspruch 12, wobei ein elastisches Element, das die stärkste Kontraktionskraft unter den elastischen Elementen aufweist, die auf der Längsaußenseite der absorbierenden Haupteinheit (1) von dem schwach haftenden Abschnitt (81) aus angeordnet sind, an einer Position angeordnet ist, die dem schwach haftenden Abschnitt (81) am nächsten liegt.

14. Absorbierender Artikel nach einem der Ansprüche 1 bis 13, wobei die Randabdeckfolie (8) an einer Längsinnenseite der absorbierenden Haupteinheit (1) einen zurückgefalteten Endabschnitt (89) aufweist und der zurückgefaltete Endabschnitt (89) in der Querrichtung (X) benachbart an beiden Seiten des schwach haftenden Abschnitts (81) befestigt ist.

15. Absorbierender Artikel nach Anspruch 14, wobei ein elastisches Element, das es der Randabdeckfolie (8) ermöglicht, sich in der Querrichtung (X) auszudehnen und zusammenzuziehen, zwischen dem zurückgefalteten Endabschnitt (89) und dem schwach haftenden Abschnitt (81) angeordnet ist.

## Revendications

1. Article absorbant, comprenant une unité principale absorbante (1) s'étendant dans le sens de la longueur, et une couverture extérieure (2) disposée sur une face superficielle n'entrant pas en contact avec la peau de l'unité principale absorbante (1), où
l'unité principale absorbante (1) a une direction longitudinale (Y) correspondant à la direction de liaison d'un côté avant, d'un côté d'entrejambe et d'un côté arrière, et une direction transversale (X) perpendiculaire à la direction longitudinale (Y) ;
une feuille de couverture de bord (8) couvrant le pourtour d'une partie d'extrémité longitudinale (15) de l'unité principale absorbante (1) dans la direction transversale (X) est disposée sur une face superficielle de l'unité principale absorbante (1) en contact avec la peau ; et
la feuille de couverture de bord (8) présente une partie à faible adhérence (81) et une partie à forte adhérence (82) avec l'unité principale absorbante (1),
la partie à faible adhérence (81) est disposée dans une zone centrale transversale (16) de l'unité principale absorbante (1) et a une force d'adhérence de 0,8 N/50mm ou moins, et
les parties à forte adhérence (82) sont disposées de part et d'autre de la partie à faible adhérence (81) dans la direction transversale (X) et ont une force d'adhérence de 1,3 N/50mm ou plus ;
la force d'adhérence étant mesurée conformément au procédé de la description.

2. Article absorbant selon la revendication 1, où la feuille de couverture de bord (8) présente, dans une zone adjacente à la partie à faible adhérence (81) sur un côté longitudinal extérieur de l'unité principale absorbante (1), une partie longitudinale à forte adhérence (83) ayant une force d'adhérence supérieure à celle dans la partie à faible adhérence (81).

3. Article absorbant selon la revendication 1 ou la revendication 2, où la partie à faible adhérence (81) est disposée en chevauchement avec un emplacement de disposition d'un corps absorbant (5) compris dans l'unité principale absorbante (1).

4. Article absorbant selon l'une des revendications 1 à 3, où les parties à forte adhérence (82) disposées de part et d'autre de la partie à faible adhérence (81) dans la direction transversale (X) sont disposées en chevauchement avec un emplacement de disposition de composants d'une partie de réception tridimensionnelle (62) comprise dans l'unité principale absorbante (1).

5. Article absorbant selon l'une des revendications 1 à 4, où la feuille de couverture de bord (8) présente, dans une zone adjacente à la partie à faible adhérence (81) sur un côté longitudinal intérieur de l'unité principale absorbante (1), une partie facilement décollable (84) dont la force d'adhérence est inférieure à celle de la partie à faible adhérence (81).

6. Article absorbant selon la revendication 5, où la partie facilement décollable (84) est une partie non adhérente.

7. Article absorbant selon l'une des revendications 1 à 6, où des parties de fixation en forme de nervures (17) dont chacune relie la feuille de couverture de bord (8) à l'unité principale absorbante (1) sont disposées de part et d'autre de la partie à faible adhérence (81) dans la direction transversale (X), en inclinaison par rapport à la direction longitudinale (Y) de l'unité principale absorbante (1), et
une extrémité de la partie de fixation (17) placée sur un côté extérieur longitudinal de l'unité principale absorbante (1) est plus proche d'un côté central transversal de l'unité principale absorbante (1) qu'une extrémité de la partie de fixation (17) placée sur un côté intérieur longitudinal de l'unité principale absorbante (1).

8. Article absorbant selon l'une des revendications 1 à 7, où la partie à faible adhérence (81) est disposée en chevauchement avec un emplacement de disposition d'un corps absorbant (5) compris dans l'unité principale absorbante (1), et la rigidité d'un site du corps absorbant (5) disposé à un emplacement de chevauchement de la partie à faible adhérence (81) est inférieure à la rigidité d'un site du corps absorbant (5) adjacent au premier site sur un côté longitudinal intérieur de l'unité principale absorbante (1).

9. Article absorbant selon la revendication 8, où le poids de base dans le site du corps absorbant (5) disposé à l'emplacement de chevauchement de la partie à faible adhérence (81) est inférieur au poids de base dans le site du corps absorbant (5) adjacent au site susdit sur le côté longitudinal intérieur de l'unité principale absorbante (1).

10. Article absorbant selon la revendication 8, où la densité dans le site du corps absorbant (5) disposé à l'emplacement de chevauchement de la partie à faible adhérence (81) est inférieure à la densité dans le site du corps absorbant (5) adjacent au site susdit sur le côté longitudinal intérieur de l'unité principale absorbante (1).

11. Article absorbant selon la revendication 8, comprenant un site où un noyau absorbant (51) ayant un matériau absorbant constituant le corps absorbant (5) n'est pas disposé dans le site du corps absorbant (5) disposé à l'emplacement de chevauchement de la partie à faible adhérence (81).

12. Article absorbant selon l'une des revendications 1 à 11, où un élément élastique permettant à la feuille de couverture de bord (8) de se dilater et de se contracter dans la direction transversale n'est pas disposé dans une zone correspondant à la partie à faible adhérence (81) de l'article absorbant, et où l'élément élastique permettant à la feuille de couverture de bord (8) de se dilater et de se contracter dans la direction transversale (X) est disposé sur un côté longitudinal extérieur de l'unité principale absorbante (1) à partir de la partie à faible adhérence (81).

13. Article absorbant selon la revendication 12, où un élément élastique ayant la force de contraction maximale parmi les éléments élastiques disposés sur le côté longitudinal extérieur de l'unité principale absorbante (1) à partir de la partie à faible adhérence (81) est disposé à l'emplacement le plus proche de la partie à faible adhérence (81).

14. Article absorbant selon l'une des revendications 1 à 13, où la feuille de couverture de bord (8) présente, sur un côté longitudinal intérieur de l'unité principale absorbante (1), une partie d'extrémité arrière de pliage (89), et la partie d'extrémité arrière de pliage (89) est fixée de part et d'autre de la partie à faible adhérence (81) dans la direction transversale (X).

15. Article absorbant selon la revendication 14, où un élément élastique permettant à la feuille de couverture de bord (8) de se dilater et de se contracter dans la direction transversale (X) est disposé entre la partie d'extrémité arrière de pliage (89) et la partie à faible adhérence (81).
